Europäisches Patentamt

**European Patent Office**

Office européen des brevets

(11) Veröffentlichungsnummer: **0 329 006**
**A2**

(12) **EUROPÄISCHE PATENTANMELDUNG**

(21) Anmeldenummer: **89102173.5**

(22) Anmeldetag: **08.02.89**

(51) Int. Cl.⁴: **G01N 13/00**

(30) Priorität: **15.02.88 DE 3804688**

(43) Veröffentlichungstag der Anmeldung:
**23.08.89 Patentblatt 89/34**

(84) Benannte Vertragsstaaten:
**CH DE FR IT LI NL**

(71) Anmelder: **Erweka Apparatebau GmbH**
**Ottostrasse 20-22**
**D-6056 Heusenstamm(DE)**

(72) Erfinder: **Schneider, Ortwin**
**Arheilgerstrasse 39**
**D-6108 Weiterstadt(DE)**

(74) Vertreter: **Kirschner, Klaus Dieter et al**
**Patentanwälte Herrmann-Trentepohl**
**Kirschner Grosse Bockhorni Forstenrieder**
**Allee 59**
**D-8000 München 71(DE)**

(54) **Zerfallstestgerät.**

㊅ Das Zerfallstestgerät zum Bestimmen des Zerfallsverhaltens fester Bestandteile, insbesondere Tabletten, in einer Flüssigkeit enthält eine Trageinrichtung mit einem auf- und abbeweglich gelagerten Träger für ein Behältnis, das die festen Bestandteile enthält, ein Flüssigkeitsbad, in das das Behältnis eintauchbar ist, eine von einem Motor angetriebene erste Hubvorrichtung, die im Normalbetrieb den Träger und damit das Behältnis in der Flüssigkeit auf- und abbewegt, und eine zweite Hubvorrichtung, die nach der Beendigung eines Tests das Behältnis aus der Flüssigkeit heraushebt. Die erste Hubvorrichtung umfaßt einen Hubarm, der vom Motor über eine Antriebswelle angetrieben wird, und eine in einem vorgegebenen Abstand von dieser angeordneten ersten Rolle, auf der die Trageinrichtung lastet. Die zweite Hubvorrichtung enthält eine aus dem Hubarm herausschiebbare zweite Rolle, die in einem größeren radialen Abstand von der Antriebswelle angeordnet ist und auf der die Trageinrichtung nach dem Herausschieben lastet, um einen großen Hub auszuführen.

Fig.2

## Zerfallstestgerät

Die Erfindung betrifft ein Zerfallstestgerät zur Bestimmung des Zerfallsverhaltens fester Bestandteile, insbesondere Tabletten, in Flüssigkeiten gemäß dem Oberbegriff des Patentanspruchs 1.

Ein derartiges Zerfallstestgerät ist sein Jahren auf dem Markt. Zur Durchführung eines Tests ist es erforderlich, daß das Behältnis in dem Flüssigkeitsbad auf- und abbewegt wird, um definierte Verhältnisse beim Zerfallsvorgang zu haben, und um bei Tabletten die Verhältnisse zu simulieren, denen die Tablette bei ihrer Auflösung im Körper des Patienten unterworfen ist. Um festzustellen, wie weit eine Tablette beispielsweise innerhalb von zehn Minuten bei kontinuierlicher Bewegung in der Flüssigkeit zerfällt, hat man bisher die Betriebsdauer des Betriebsmotors auf zehn Minuten eingestellt, so daß der Motor nach Ablauf dieser Zeit stehenbleibt. Diesen Zeitpunkt hat man mehr oder weniger genau überwacht und das Behältnis dann aus der Flüssigkeit von Hand entnommen. Bei dieser Praxis läßt es sich nicht vermeiden, daß die Tabletten noch unbeabsichtigt noch einige Zeit in der Flüssigkeit verbleiben, bevor sie herausgehoben werden, wenn nämlich der Laborant das Ende des Tests nicht sofort feststellt.

Aus dem DE-GM 86 15 404.4 ist bereits ein Zerfallstestgerät bekannt, das eine erste von einem Motor angetriebene Hubeinrichtung aufweist, mittels derer eine Trageinrichtung auf-und abbewegt wird, an der ein mit den Tabletten versehenes Behältnis befestigt ist und in der Flüssigkeit damit ebenfalls auf- und abbewegt wird. Dieses bekannte Zerfallstestgerät enthält eine zweite Hubvorrichtung, die bei einer Betätigung das Behältnis aus der Flüssigkeit heraushebt. Nach dem Herausheben wird der Motor abgeschaltet und damit eine zuverlässige Arbeitsweise erreicht, da das Behältnis selbsttätig zu einer genau vorgegebenen Zeit aus der Flüssigkeit herausgenommen wird. Diese zweite Hubvorrichtung erfordert jedoch einen verhältnismäßig großen Aufwand.

Der Erfindung liegt daher die Aufgabe zugrunde, ein Zerfallstestgerät anzugeben, mittels dessen das Behältnis selbsttätig aus der Flüssigkeit herausnehmbar ist und das dennoch einen geringen Aufwand erfordert.

Erfindungsgemäß wird die Aufgabe bei dem Zerfallstestgerät der eingangs genannten Art durch das im kennzeichnenden Teil des Patentanspruchs 1 angegebene Merkmal gelöst.

Das erfindungsgemäße Zerfallstestgerät hat den Vorteil, daß es nur wenige bewegte Teile für die zweite Hubvorrichtung erfordert und daß es damit einfach herzustellen ist und zuverlässig arbeitet.

Weiterbildungen der Erfindung ergeben sich aus den Unteransprüchen.

Ein Ausführungsbeispiel des erfindungsgemäßen Zerfallstestgeräts wird im Folgenden anhand der beiligenden Zeichnungen beschrieben. Es zeigen:

Fig. 1 eine Darstellung des Zerfallstestgeräts in einer teilweise geschnittenen ersten Seitenansicht;

Fig. 2 eine Darstellung des Zerfallstestgeräts in einer teilweise geschnittenen zweiten Seitenansicht;

Fig. 3a bis 3d eine Darstellung einer ersten Hubvorrichtung in verschiedenen Stellungen beim Auf- und Abbewegen einer Trageinrichtung;

Fig. 4a bis 4c eine Darstellung einer zweiten Hubvorrichtung in verschiedenen Stellungen beim Herausheben eines Behältnisses aus einem Flüssigkeitsbad mittels der Trageinrichtung;

Fig. 5a und 5b eine Darstellung eines Hubarms; und

Fig. 6a und 6b eine Darstellung eines Keils.

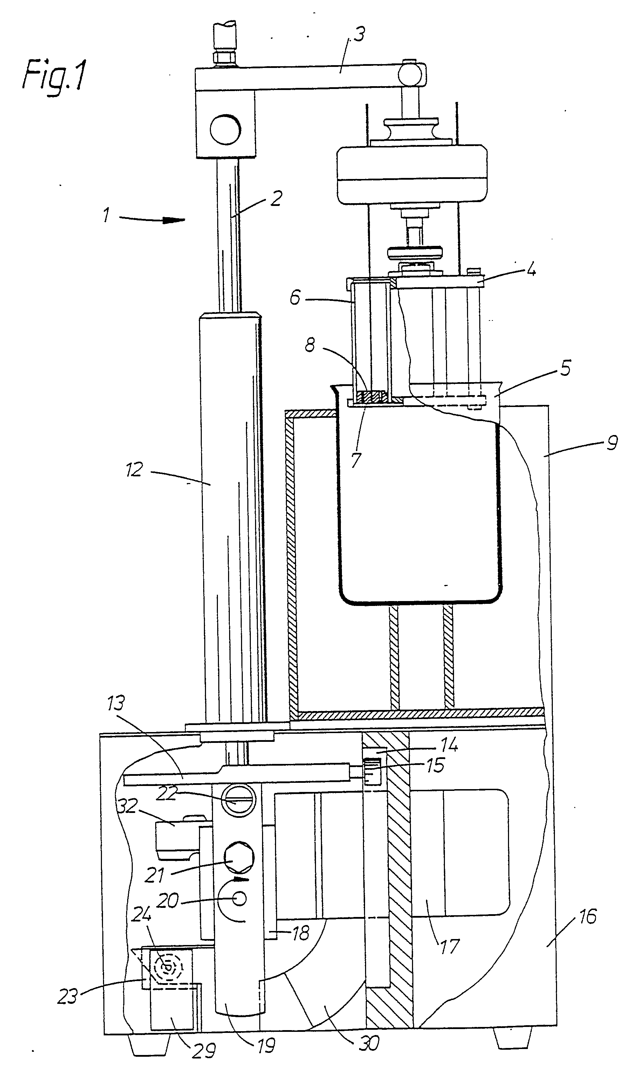

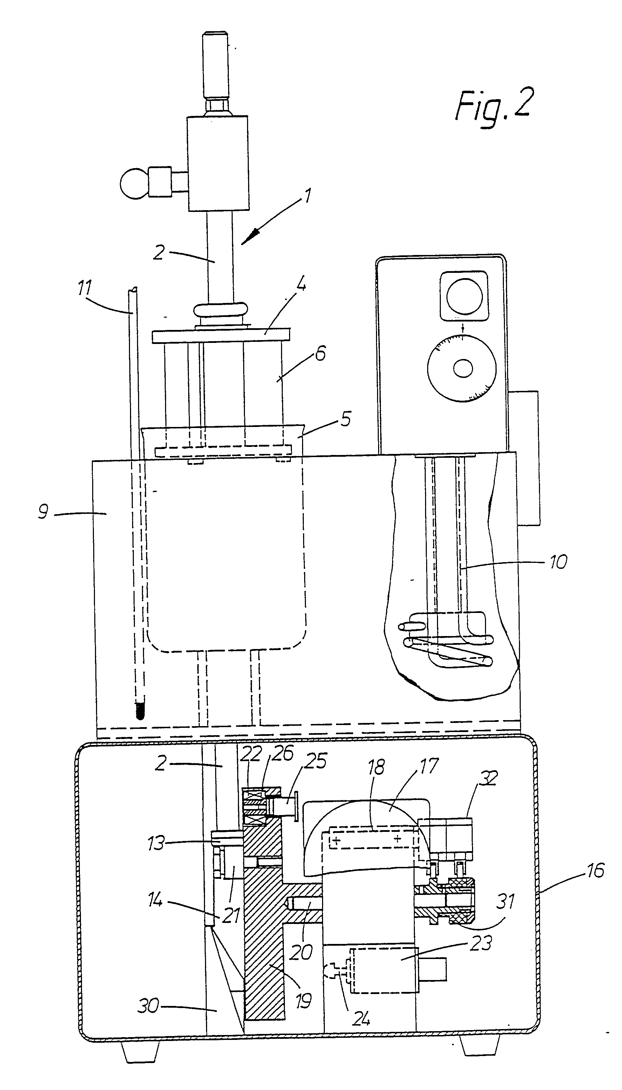

Das in den Fig. 1 und 2 dargestellte Zerfallstestgerät enthält eine Trageinrichtung 1 mit einem auf- und abbeweglichen Träger 2, an dem über einen Galgen 3 ein Behältnis 4 aufgehängt ist. Dieses Behältnis 4 enthält Tabletten, deren Zerfallsverhalten in einer in einem Becher 5 enthaltenen Flüssigkeit bestimmt werden soll. In dem Behältnis 4 sind unten offene Glasröhrchen 6 angeordnet, die auf dem als Sieb ausgebildeten Boden 7 des Behältnisses 4 stehen. In jedem Glasröhrchen 6 liegt eine Tablette, die durch eine Scheibe 8 beschwert ist.

Der Zerfallstest erfolgt in der Weise, daß das Behältnis 4 in die Flüssigkeit abgesenkt und darin in vertikaler Richtung hin- und herbewegt wird. Es wird dann im Wesentlichen be stimmt, in welcher Zeit sich die Tabletten in der Flüssigkeit auflösen. Die Flüssigkeit wird dabei durch Eintauchen des Bechers 5 in eine in einem Behälter 9 untergebrachte Flüssigkeit auf einer vorgegebenen Temperatur gehalten. Das Heizen dieser Flüssigkeit erfolgt dabei mittels einer Heizeinrichtung 10, die thermostatisch gesteuert wird. Eine Bedienperson kann die Temperatur an einem Thermometer 11 ablesen. Nach Beendigung des Tests wird das Behältnis 4 wieder aus der Flüssigkeit herausgehoben.

Der Träger 2 der Trageinrichtung 1 ist in einem Führungsrohr 12 auf- und abbeweglich gelagert und hat an seinem unteren Ende einen quer zur Längsrichtung des Trägers 2 angeordneten Balken 13. Um zu verhindern, daß sich der Träger 2 in dem Führungsrohr 12 verdreht, ist eine Führungsschiene 14 vorgesehen, die eine Ausnehmung aufweist, in der eine mit dem Balken 13 verbundene Rolle 15 läuft. Das Führungsrohr 12 und die Führungsschiene 14 sind an einem Sockelteil 16 ortsfest angeordnet.

Bei der Durchführung eines Tests soll das Behältnis 4 in der Flüssigkeit zwischen einer oberen und einer unteren Lage hin-und herbewegt werden und am Ende des Tests soll das Behältnis 4 aus der Flüssigkeit herausgehoben werden. Dazu sind eine ersten und eine zweite Hubvorrichtung vorgesehen, die in den Fig. 1 und 2 gezeigt sind.

Die erste Hubvorrichtung, die zur Auf- und Abbewegung des Behältnisses 4 in der Flüssigkeit dient, weist einen von einem Motor 17 über ein Getriebe 18 angetriebenen Hubarm 19 auf, der mittig mit einer vom Getriebe 18 angetriebenen Antriebswelle 20 verbunden ist. In einem vorgegebenen ersten Abstand von der Antriebswelle 20 ist an dem Hubarm 19 eine Rolle 21 drehbar befestigt, auf der der Balken 13 lastet. Bei einer Drehung des Hubarms 19 und der Antriebswelle 20 bewegt sich die Rolle 21 auf einer Kreisbahn um die Antriebswelle 20. Da der Balken 13 auf der Rolle 21 lastet, wird dieser auf- und abbewegt und mit ihm wird über die Trageinrichtung 1 das Behältnis 4 in der Flüssigkeit auf- und abbewegt.

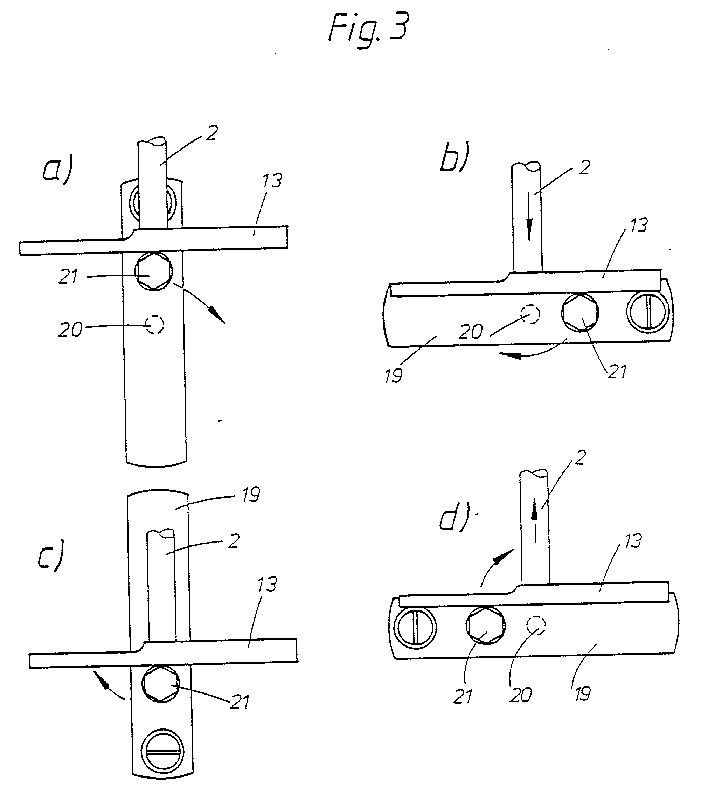

Die Fig. 3a bis 3d zeigen die erste Hubvorrichtung in verschiedenen Stellungen bei der Drehung der Antriebswelle 20. Bei der Darstellung in Fig. 3a befindet sich der Balken 13 in seiner oberen Stellung. In Fig. 3b hat sich der Hubarm 19 um 90° im Uhrzeigersinn gedreht und der Balken 13 befindet sich in einer mittleren Stellung. Bei einer weiteren Drehung um 90° befindet sich der Balken 13 in seiner untersten Stellung, die in Fig. 3c dargestellt ist. Nach einer weiteren Drehung um 90° befindet sich der Balken 13 wieder in einer mittleren Stellung, die derjenigen von Fig. 3b entspricht und in Fig. 3d dargestellt ist. Nach einer weiteren Drehung um 90° beginnt ein neuer Zyklus, ausgehend von der Stellung, die in Fig. 3a dargestellt ist.

Mit der Bewegung des Balkens 13 entsprechend Fig. 3 wird auch die Trageinrichtung 1 und damit das Behältnis 4 auf- und abbewegt, so daß die Tabletten in der Flüssigkeit hin- und herbewegt werden.

Die zweite Hubvorrichtung zum selbsttätigen Herausheben des Behältnisses 4 aus der Flüssigkeit wird aus einer weiteren Rolle 22 gebildet, die an dem Hubarm 19 in einem größeren Abstand von der Antriebswelle 20 als die Rolle 21 angeordnet ist und außerdem herausschiebbar ausgebildet ist. Das Herausheben erfolgt am Ende eines Tests, nachdem eine vorgegebene Zeitdauer vergangen ist und/oder eine vorgegebene Anzahl von Hin- und Herbewegungen in der Flüssigkeit ausgeführt worden ist. Die zweite Hubvorrichtung wird dann durch einen Elektromagneten 23 betätigt, dessen Anker 24 in einer geeigneten Stellung des Hubarms 19 von hinten über eine federnde Zunge 29 gegen einen Stößel 25 und über diesen gegen die Lagerung 26 der Rolle 22 stößt und diese nach vorne aus dem Hubarm 19 herausschiebt. Das Herausschieben erfolgt insbesondere dann, wenn bei einer Drehung des Hubarms 19 die Rolle 22 unterhalb der Rolle 21 liegt, wird es beispielsweise nach der in Fig. 3b und vor der in Fig. 3d dargestellten Stellung der Fall ist.

Das Zurückschieben der Rolle 22 in den Hubarm 19 erfolgt unter Verwendung eines Keils 30, der dem Elektromagneten 12 gegenüberliegend im unteren Bereich des Sockelteils 16 angeordnet ist. Bei der Drehung des Hubarms 19 schlägt die herausgeschobene Rolle 22 gegen den Keil 30, so daß sie während der weiteren Drehung durch den Keil 30 in den Hubarm 19 zurückgeschoben wird.

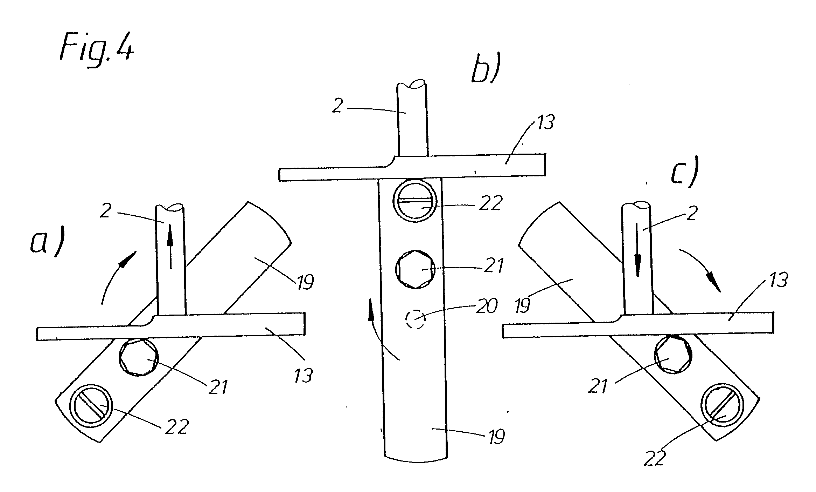

Bei der Darstellung in Fig. 4a lastet der Balken 13 wie bei der normalen Hin- und Herbewegung des Behältnisses 4 in der Flüssigkeit während der Drehung des Hubarms 19 auf der Rolle 21 und in dieser Stellung wird der Elektromagnet 23 betätigt, so daß sein Anker 24 über die federnde Zunge 29 von hinten gegen den Stößel 25 schlägt und die Rolle 22 aus dem Hubarm 19 herausschiebt. Nach der horizontalen Stellung des Hubarms 19 entsprechend Fig. 3d übernimmt dann die Rolle 22 das Abstützen des Balkens 13, so daß dessen Hub größer wird. Bei der in Fig. 4b dargestellten Stellung hat der Balken 13 seine oberste Stellung erreicht und in dieser Stellung ist das Behältnis 4 über die Trageinrichtung 1 aus der Flüssigkeit herausgehoben. In dieser Stellung wird der Motor 17 angehalten und der Test ist beendet.

Bei einem erneuten Starten des Motors 17 wird der Hubarm 19 wieder gedreht und, nachdem er die in Fig. 3b dargestellte Stellung verlassen hat, übernimmt wieder die Rolle 21 das Abstützen des Balkens 13. In der in Fig. 4c dargestellten Stellung trifft die herausgeschobene Rolle 22 auf den Keil 30, so daß sie bei einer weiteren Drehung des Hubarms 19 wieder in diesen zurückgeschoben wird und eine Bewegung entsprechend den Fig. 3a bis 3d wieder durchgeführt wird, d.h., das Behältnis 4 wird in der Flüssigkeit so lange auf- und abbewegt, bis der Elektromagnet 23 wieder betätigt wird und die Rolle 22 das Herausheben des Behältnisses 4 aus der Flüssigkeit über die Trageinrichtung 1 bewirkt.

Das Ansteuern des Elektromagneten 23 und das anschließende genaue Anhalten des Motors 17 in der obersten Stellung des Balkens 13 erfolgt durch Schalter 32, die durch Nockenscheiben 31 gesteuert werden, die am rückwärtigen Ende der Antriebswelle 20 angeordnet sind.

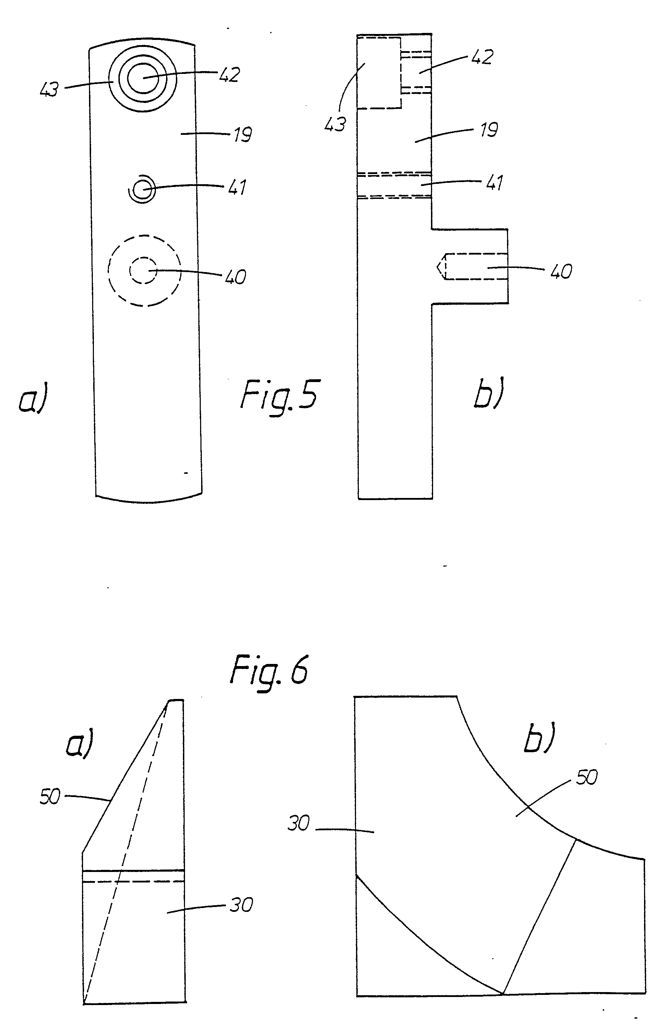

Der in den Fig. 5a und 5b dargestellte Hubarm 19 enthält eine Bohrung 40 für die Antriebswelle 20 sowie ein Gewinde 41 für die Befestigung der Rolle 21 der ersten Hubvorrichtung. Weiterhin enthält der Hubarm 19 eine Bohrung 42 für die Lagerung der Rolle 22 durch das Lager 26 und zur Führung des Stößels 25 sowie eine Ausnehmung 43 für die Aufnahme der Rolle 22 im zurückgezogenen Zustand.

Die Fig. 6a und 6b zeigen den Keil 30 in einer Seitenansicht bzw. einer Draufsicht. Der Keil 30 ist am Boden des Sockelteils 16 befestigt und weist eine Auflauffläche 50 auf, auf die die Rolle 22 im herausgezogenen Zustand bei einer Drehung des Hubarms 19 auftrifft und die durch ihre Schräge gegen die Umfangsrichtung bei einer weiteren Drehung des Hubarms 19 die herausgeschobene Rolle 22 wieder in ihre Ausnehmung 43 zurückschiebt, wenn nur eine Auf- und Abbewegung der Trageinrichtung 1 durchgeführt werden soll.

**Ansprüche**

1. Zerfallstestgerät zum Bestimmen des Zerfallsverhaltens fester Bestandteile, insbesondere Tabletten, in Flüssigkeiten, wobei das Zerfallstestgerät eine Trageinrichtung mit einem auf- und abbeweglich gelagerten Träger für ein Behältnis, das die festen Bestandteile enthält, ein Flüssigkeitsbad, in das das Behältnis eintauchbar ist, eine erste Hubvorrichtung, die eine an einem Hubarm in einem vorgegebenen radialen Abstand von einer motorisch angetriebenen Antriebswelle angeordnete Rolle umfaßt, auf der sich der Träger abstützt, der bei einer Drehung des Hubarms das Behältnis in der Flüssigkeit auf- und abbewegt, und eine zweite Hubvorrichtung aufweist, die bei Betätigung das Behältnis aus dem Flüssigkeitsbad heraushebt, **dadurch gekennzeichnet,** daß die zweite Hubvorrichtung eine betätigbare zweite Rolle (22) umfaßt, die in einem größeren radialen Abstand von der Antriebswelle (20) am Hubarm (19) angeordnet ist als die erste Rolle (21) und die nach ihrer Betätigung den Träger (2) für das Herausheben des Behältnisses (4) aus der Flüssigkeit abstützt.

2. Zerfallstestgerät nach Anspruch 1, **dadurch gekennzeichnet,** daß die zweite Rolle (22) in derselben radialen Richtung wie die erste Rolle (21) am Hubarm (19) angeordnet ist.

3. Zerfallstestgerät nach Anspruch 1 oder 2, **dadurch gekennzeichnet,** daß die Betätigung der zweiten Rolle (22) durch ihr Herausschieben aus dem Hubarm (19) in axialer Richtung erfolgt.

4. Zerfallstestgerät nach Anspruch 3, **dadurch gekennzeichnet,** daß das Herausschieben der zweiten Rolle (22) durch einen Elektromagneten (23) erfolgt.

5. Zerfallstestgerät nach Anspruch 4, **dadurch gekennzeichnet,** daß der Anker (24) des Elektromagneten (23) von hinten gegen ein in dem Hubarm (19) gelagertes Lager (26) der zweiten Rolle (22) schlägt.

6. Zerfallstestgerät nach Anspruch 5, **dadurch gekennzeichnet,** daß der Anker (24) über einen Stößel (25) gegen das Lager schlägt.

7. Zerfallstestgerät nach einem der Ansprüche 3 bis 6, **dadurch gekennzeichnet,** daß das Zurückschieben der zweiten Rolle (22) in den Hubarm (19) durch einen Keil (30) erfolgt, auf dessen schräger Fläche die zweite Rolle (22) bei der Drehung des Hubarms (19) auftrifft.

8. Zerfallstestgerät nach Anspruch 7, **dadurch gekennzeichnet,** daß der Keil (30) und der Magnet (23) unterhalb der Antriebswelle (20) angeordnet sind.

9. Zerfallstestgerät nach einem der Ansprüche 4 bis 8, **dadurch gekennzeichnet,** daß die Ansteuerung des Elektromagneten (23) und/oder das Abschalten des Motors (17) durch über eine Nockenscheibe (31) betätigbare Schalter (32) erfolgt.

Fig.1

EP 0 329 006 A2

*Fig.2*

Fig. 3

Fig.4

a)

43 — 42

19

41

40

Fig.5

b)

43

42

19

41

40

Fig. 6

a)

50

30

b)

50

30